# FASCICULE DE BREVET EUROPEEN

(11) **EP 3 165 915 B1**
(45) Date de publication et mention de la délivrance du brevet: **17.10.2018**
(21) Numéro de dépôt: 16206006.5
(22) Date de dépôt: 28.09.2012
(51) Int. Cl.: G01N 29/02, G01N 29/036, G01N 33/22, G01N 21/64

(54) **UTILISATION DE NANOPARTICULES D'OXYDES DE METAUX DE TRANSITION COMME MATERIAUX SENSIBLES DANS DES CAPTEURS CHIMIQUES POUR LA DETECTION OU LE DOSAGE DE VAPEURS DE MOLECULES CIBLES**
EINSATZ VON NANOPARTIKELN AUS ÜBERGANGSMETALLOXIDEN ALS SENSITIV MATERIALIEN FÜR CHEMISCHE SENSOREN ZUR DETEKTION ODER DOSIERUNG VON ZIELMOLEKÜLDÄMPFEN
USE OF NANOPARTICLES OF TRANSITION-METAL OXIDES AS SENSITIVE MATERIALS IN CHEMICAL SENSORS FOR DETECTING OR ASSAYING TARGET MOLECULE VAPOURS

(30) Priorité: 29.09.2011 FR 1158755
(43) Date de publication de la demande: 10.05.2017
(62) Demande divisionnaire de: 12762649.7
(73) Titulaire: Commissariat à l'Energie Atomique et aux Energies Alternatives, 75015 Paris (FR)
(72) Inventeur: PEREIRA, Franck, 37260 MONTS (FR); OUDOT, Natacha, 33400 TALENCE (FR)
(74) Mandataire: Brevalex

(56) Documents cités:
- FR-A1- 2 859 393
- GE ET AL: "Preparation and gas-sensing properties of Ce-doped ZnO thin-film sensors by dip-coating", MATERIALS SCIENCE AND ENGINEERING B, ELSEVIER SEQUOIA, LAUSANNE, CH, vol. 137, no. 1-3, 7 février 2007 (2007-02-07), pages 53-58, XP005878345, ISSN: 0921-5107, DOI: 10.1016/J.MSEB.2006.10.006
- ZHOU Q ET AL: "An ethanol gas sensor using energy transfer cataluminescence on nanosized YVO4:Eu<3+> surface", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, vol. 144, no. 1, 29 janvier 2010 (2010-01-29), pages 192-197, XP026862016, ISSN: 0925-4005 [extrait le 2009-10-29]

## Description

### DOMAINE TECHNIQUE

La présente invention concerne l'utilisation de nanoparticules inorganiques d'oxydes de métaux de transition, plus précisément de nanoparticules d'oxyde d'au moins un métal de transition dopé par une terre rare, lesdites nanoparticules étant obtenues par un procédé sol-gel, en tant que matériaux sensibles dans des capteurs chimiques destinés à détecter ou à doser des vapeurs de composés ou molécules cibles.

Ces composés ou molécules cibles sont notamment des polluants atmosphériques, des explosifs ou des précurseurs d'explosifs.

L'invention a également trait aux capteurs chimiques comprenant lesdites nanoparticules comme matériaux sensibles.

L'invention se reporte plus particulièrement à la détection et au dosage de peroxydes et notamment du peroxyde d'hydrogène.

Le peroxyde d'hydrogène est un composé à partir duquel il est possible de préparer artisanalement des explosifs comme le triperoxyde de triacétone (TATP) ou le triperoxyde d'hexaméthylène diamine (HMTD), et le peroxyde d'hydrogène est aussi un produit de décomposition de ces explosifs, de ce fait l'invention trouve notamment son application dans la lutte contre le terrorisme.

Les peroxydes sont, par ailleurs, des composés très instables qui se décomposent facilement en libérant, pour un certain nombre d'entre eux, des vapeurs inflammables, de ce fait l'invention trouve également son application dans la surveillance, à des fins sécuritaires, de locaux dans lesquels sont fabriqués, stockés et/ou utilisés des peroxydes ou des composés peroxydables, c'est-à-dire des composés qui, initialement, ne sont pas des peroxydes mais qui sont susceptibles de se transformer au moins partiellement en peroxydes suite à un phénomène d'auto-oxydation, encore appelé peroxydation, ainsi que dans la surveillance de la pollution atmosphérique.

### ÉTAT DE LA TECHNIQUE ANTÉRIEURE

Les peroxydes sont des composés chimiques qui comprennent un ou plusieurs groupes -O-O- et qui ont, de ce fait, un fort pouvoir oxydant.

Ils sont donc couramment utilisés comme agents blanchissants, notamment dans l'industrie textile, pour le blanchiment de fibres naturelles comme les fibres de coton, et dans l'industrie papetière, pour le blanchiment de la pâte à papier. Ils sont également couramment utilisés comme initiateurs, promoteurs ou catalyseurs dans des procédés de polymérisation radicalaire, ainsi que comme agents de réticulation ou de vulcanisation dans l'industrie des matières plastiques.

A cela, il faut ajouter des utilisations propres à chaque peroxyde.

Ainsi, par exemple, le peroxyde d'hydrogène, de formule H₂O₂, encore connu sous le nom d'eau oxygénée, est employé comme :
- agent de désinfection ;
- agent de stérilisation, notamment dans l'industrie alimentaire où, vaporisé à haute température, il sert à stériliser les emballages composites juste avant l'incorporation des produits alimentaires, et dans l'industrie médicale où il sert à stériliser des dispositifs médicaux thermosensibles et, donc, non stérilisables par la chaleur ;
- agent de traitement d'eaux résiduaires domestiques ou industrielles ; et comme
- agent de traitement d'effluents gazeux.

Il se trouve que le peroxyde d'hydrogène est aussi susceptible d'être utilisé pour fabriquer de manière artisanale des peroxydes organiques explosifs comme le TATP ou le HMTD et que ces explosifs donnent, lorsqu'ils se décomposent, du peroxyde d'hydrogène.

Les peroxydes organiques tels que le TATP ou le HMTD sont des composés qui peuvent être aisément synthétisés à partir de précurseurs facilement accessibles et qui sont aussi puissants que le TNT.

L'instabilité de ces composés les rend inutilisables pour des applications militaires, par contre ces composés sont régulièrement impliqués dans des actes terroristes du fait qu'ils ne sont pas détectables par les capteurs actuellement mis en place qui sont sensibles aux explosifs nitrés.

Il se trouve également que les peroxydes ont la particularité d'être des composés très instables et de se décomposer en libérant, pour un certain nombre d'entre eux, des vapeurs inflammables.

Il est donc hautement souhaitable de pouvoir disposer de dispositifs permettant de détecter de manière fiable mais néanmoins rapide la présence d'explosifs artisanaux ou de leurs précurseurs, et en particulier la présence de peroxydes, notamment lorsqu'ils sont à l'état de vapeurs, que ce soit pour déjouer une menace terroriste ou pour prévenir tout risque d'accident dans des locaux où sont fabriqués, stockés et/ou utilisés des peroxydes ou des composés peroxydables.

En particulier, le peroxyde d'hydrogène étant à la fois un précurseur des peroxydes organiques et un produit de dégradation de ces composés, il paraît primordial d'être en mesure de le détecter.

Ces composés explosifs et leurs précurseurs, y compris H₂O₂, présentent généralement de très faibles tensions de vapeur, en conséquence les composés et précurseurs doivent pouvoir être détectés à l'état de traces.

En outre, l'application visée étant notamment le contrôle des bagages, les techniques de détection mises en oeuvre doivent être compatibles avec les transports de masse.

Les méthodes actuellement utilisées pour détecter des vapeurs de composés, par exemple de peroxydes, entrant notamment dans la composition d'explosifs, mettent en oeuvre des dispositifs d'analyses comme des spectromètres à mobilité ionique, des spectromètres de masse, des analyseurs infrarouge ou des techniques de chromatographie couplées à un spectromètre.

Ces méthodes donnent d'une manière générale, des résultats satisfaisants en termes de sensibilité et de fiabilité, ce qui est primordial en matière de détection d'explosifs compte tenu de la très faible concentration en vapeurs dans l'atmosphère.

Cependant, ces méthodes présentent également de nombreux inconvénients comme leur coût élevé, l'encombrement important et la consommation d'énergie élevée des dispositifs qu'elles utilisent, et leur mise en oeuvre complexe, qui rend indispensable la présence d'un spécialiste pour interpréter les signaux obtenus.

Ces méthodes ne peuvent en outre être utilisées en tout lieu, et notamment en des lieux et sites difficiles d'accès car les dispositifs qu'elles utilisent ne peuvent être rendus portables et autonomes.

L'analyse peut également être effectuée en laboratoire par les dispositifs mentionnés plus haut après avoir effectué des prélèvements d'échantillons sur site mais cette méthode ne permet pas d'obtenir une réponse rapide.

Le recours aux « chiens renifleurs » ou autres animaux dressés et entraînés pour la détection de vapeurs présente l'inconvénient de nécessiter une longue période de formation des chiens et de leurs maîtres et exclut des interventions continues et prolongées en raison d'une durée limitée d'attention de ces animaux. De plus, cette méthode nécessite elle aussi la présence d'un spécialiste comme un maître chien par exemple.

Depuis un certain nombre d'années, le développement de capteurs capables de détecter en temps réel des espèces, composés, molécules chimiques, notamment des espèces gazeuses, est en plein essor.

Le fonctionnement de ces capteurs est basé sur l'utilisation d'un matériau sensible, c'est-à-dire d'un matériau dont au moins une propriété physique et/ou chimique est modifiée au contact des espèces, composés, molécules chimiques recherchés encore appelés espèces, composés, molécules cibles.

Ce matériau sensible est relié à un système apte à mesurer instantanément toute variation de cette propriété physique et/ou chimique pour mettre ainsi en évidence la présence des espèces, composés, molécules chimiques recherchés.

Plus précisément, le principe de fonctionnement de ces capteurs chimiques repose sur l'interaction hétérogène de la molécule cible, par exemple sous forme de gaz, avec un matériau sensible, notamment sous forme de couche mince solide ou en solution pulvérisée, qui engendre la variation d'au moins une propriété physico-chimique de ce matériau.

Cette variation peut être de nature électrique, optique, mécanique, magnétique. Il peut s'agir d'une évolution de la masse du matériau sensible, due à l'adsorption des molécules notamment des molécules gazeuses recherchées, à la surface du matériau sensible.

La variation de la propriété du matériau sensible est alors mesurée en temps réel puis transformée par un système de transduction adapté en un signal électrique exploitable.

Ce signal est enfin transmis au dispositif de mesure où il subit un traitement algorithmique adéquat afin d'indiquer la présence ou non des molécules recherchées, par exemple la présence ou non des molécules gazeuses recherchées dans l'atmosphère.

Les avantages des capteurs chimiques, notamment par rapport aux méthodes précitées sont multiples : instantanéité des résultats, détection en temps réel, possibilité de miniaturisation et, donc, portabilité, maniabilité et autonomie importante, faibles coûts de fabrication et d'exploitation, simplicité d'utilisation etc.

Toutefois, il est évident que leurs performances sont extrêmement variables selon la nature du matériau sensible utilisé.

Ainsi, pour la détection des peroxydes et en particulier du peroxyde d'hydrogène, divers matériaux sensibles ont été envisagés.

Une approche habituelle pour détecter le peroxyde d'hydrogène H₂O₂ consiste à mesurer le courant quand le peroxyde est oxydé à une électrode solide.

Afin d'améliorer la réponse, plusieurs études ont consisté à revêtir la surface de l'électrode avec des substances présentant une activité catalytique comme des oxydes de métal de transition [1], des enzymes [2], du bleu de Prusse [3] ou de la phtalocyanine de métal [4].

Une autre approche consiste à utiliser des matériaux conducteurs tels que les nanotubes de carbone dopés avec différents éléments comme le platine [5] ou un complexe EDTA/fer [6] par exemple pour améliorer les propriétés électrocatalytiques.

Ces techniques analytiques permettent de mesurer directement la concentration en H₂O₂ présent dans une solution, ou bien la concentration en H₂O₂ généré lorsque du TATP ou du HMTD sont traités par les ultraviolets ou par un rayon laser. Il est ainsi possible de réaliser une détection indirecte de ces espèces.

Les peroxydes organiques peuvent également être décomposés en milieu acide [7] pour former du H₂O₂.

Si les techniques décrites plus haut sont utiles en milieu liquide, elles apparaissent pour la plupart peu adaptées à la détection de vapeurs de peroxydes.

Seules les phtalocyanines [4] déposées sous la forme de films de 50 nm d'épaisseur, permettent la détection du H₂O₂ sous forme de vapeurs en une dizaine de minutes.

D'autres composés, molécules organiques, qu'il s'agisse de colorants ou de composés luminescents, peuvent être déposés sous la forme de films sur des substrats, pour former ainsi des bandes tests capables de détecter les peroxydes, dont la présence provoque une diminution de l'intensité lumineuse émise par ces molécules.

Des exemples de telles molécules sont le vert de lissamine (colorant organique) encapsulé dans de l'alcool polyvinylique (PVA) [8], ou le bronze d'hydrogène et de molybdène (MoHB; Mo₂0₅(OH)) [9].

Le blanchiment observé en présence d'un oxydant fort comme les peroxydes est dû à une dégradation de la molécule organique. Ces capteurs sont donc irréversibles et affichent une faible sélectivité responsable de nombreux faux positifs. De plus, les molécules organiques étant globalement peu stables sous éclairement, ces capteurs offriront une faible durée de vie et ne permettront pas un fonctionnement en continu.

Une autre méthode consiste à utiliser des molécules organiques qui produisent un signal de fluorescence lors de leur dégradation par H₂O₂. C'est le cas du poly[3',6'-bis(1,3,2-dioxaborinane)fluoran] (PolyF-1) [10], des prochélateurs [11] ou des sulfoxydes de pyrène [12].

L'avantage de cette méthode est que l'on visualise une apparition de la fluorescence, qui est bien plus facile à détecter qu'une diminution de la fluorescence.

Cependant, cette méthode conserve tous les inconvénients dus à la mise en oeuvre de molécules organiques, à savoir notamment une faible durée de vie des capteurs.

Les composés inorganiques semblent donc être mieux adaptés à la détection des peroxydes et des matériaux sensibles inorganiques ont déjà été utilisés pour détecter le H₂O₂ en solution.

Le document de Shu et *al.* [13] décrit un composite TiO₂/SiO₂ préparé par voie sol-gel. Sous une longueur d'onde d'excitation de 403 nm, la phosphorescence du composite diminue lors de l'addition d'une solution de peroxyde d'hydrogène.

De même, le document de Casanova et *al.* [14] fait mention d'une sonde luminescente pour détecter le peroxyde d'hydrogène en phase aqueuse qui utilise un matériau de type YVO₄ dopé à l'europium.

L'oxydation de l'Eu II en Eu III en présence de H₂O₂ conduit alors à une augmentation de la fluorescence.

Par ailleurs, il est communément admis que, pour la détection de composés gazeux, le matériau sensible doit présenter une grande surface spécifique afin de favoriser les interactions hétérogènes.

Ainsi, l'utilisation de films minces mésoporeux constitués de nanoparticules de silice incorporant des molécules sondes permet notamment la détection de composés nitro-aromatiques gazeux avec une grande sensibilité du fait d'une surface spécifique élevée [15]. Le document de GE et al., « Préparation and gas-sensing properties of Ce-doped ZnO thin-film sensors by dip-coating », Materials Science and Engineering B, vol. 137, 7 Février 2007, pages 53-58 décrit des nanoparticules de ZnO dopées au Cérium synthétisées par un procédé sol-gel. Des capteurs de gaz comprenant ces nanoparticules sont aussi préparés. Il ressort donc de ce qui précède que les matériaux sensibles organiques proposés pour les capteurs chimiques, notamment destinés à la détection des peroxydes, présentent l'inconvénient majeur d'une très faible robustesse car ils possèdent une très courte durée de vie lorsqu'ils sont soumis à une irradiation, en particulier lorsqu'ils sont exposés à la lumière du jour.

Les matériaux sensibles inorganiques permettent de surmonter cet inconvénient, mais bien que certains matériaux sensibles inorganiques aient été proposés pour la détection du peroxyde d'hydrogène, cette détection ne concerne que le peroxyde d'hydrogène à l'état liquide.

Il existe donc, au regard de ce qui précède, un besoin pour un matériau sensible inorganique qui permette de détecter les vapeurs de peroxydes, en d'autres termes qui soit capable de réagir à la présence de peroxydes se trouvant à l'état de vapeurs.

Il existe encore un besoin pour un matériau sensible qui, lorsqu'il est utilisé dans un capteur chimique possède toutes les caractéristiques requises pour un tel matériau comme la sensibilité (affinité du matériau sensible pour l'analyte, en l'espèce les vapeurs de peroxydes permettant sa détection sous forme de traces) associée à une rapidité de réponse, la sélectivité (sensibilité du matériau seulement à l'analyte visé), la réversibilité (capacité à revenir à l'état initial après détection) qui permettra un fonctionnement en continu, et la stabilité dans le temps et dans les conditions d'utilisation.

Le but de la présente invention est, entre autres, de répondre aux besoins et exigences énumérés plus haut.

### EXPOSÉ DE L'INVENTION

La présente invention a donc pour objet, l'utilisation de nanoparticules d'au moins un oxyde d'au moins un métal de transition dopé par une terre rare, obtenues par un procédé sol-gel, en tant que matériau sensible dans un capteur chimique pour la détection ou le dosage de vapeurs d'un ou plusieurs composé(s) ou molécule(s) cible(s), dans laquelle les nanoparticules se présentent dans le capteur sous la forme d'un sol de nanoparticules dans un solvant.

Par métal de transition, on entend tous les éléments ayant un numéro atomique de 21 à 30 ; de 39 à 48 ; et de 72 à 80.

Des métaux de transition préférés sont le vanadium et l'yttrium.

L'oxyde peut être un oxyde simple, d'un seul métal de transition, tel que le vanadium, ou bien un oxyde mixte de deux métaux de transition tels que le vanadium et l'yttrium, ou bien encore un oxyde de plus de deux métaux de transition.

Par terre rare, on entend tous les éléments ayant un numéro atomique de 57 à 71 ou lanthanides.

Des terres rares préférées sont l'europium, l'erbium ou l'ytterbium.

Par « dopé par une terre rare », on entend généralement que ladite terre rare représente de 1% à 30% en masse de la masse totale de l'oxyde.

Dans la présente, la notation VO₄ : Eu, par exemple, signifie que VO₄ est dopé par de l'europium en toutes proportions.

De la même manière, la notation YVO₄ : Eu signifie que YVO₄ est dopé par de l'europium en toutes proportions.

L'utilisation de nanoparticules d'oxyde de vanadium dopé à l'europium en tant que matériau solide pour le stockage optique de données, dans des nano-émetteurs de lumière rouge pour des lampes, ou en tant que matériau pour concentrateurs solaires, est certes mentionnée respectivement dans les documents [16], [17], et [18]. Dans ces documents, les nanoparticules ne sont jamais associées à un dispositif de transduction afin de constituer un capteur chimique de gaz capable notamment de détecter des vapeurs de peroxydes.

L'utilisation qui fait l'objet de l'invention permet de répondre aux besoins et exigences énumérés plus haut.

Avantageusement, les nanoparticules sont des nanoparticules de YₓVO₄Eu₁₋ₓ, où x a une valeur de 0 à 0,995.

De préférence, les nanoparticules sont choisies parmi les nanoparticules de VO₄Eu, c'est-à-dire que x = 0 dans la formule YₓVO₄Eu₁₋ₓ, et les nanoparticules de Y_{0,5}VO₄Eu_{0,5}, c'est-à-dire que x = 0,5 dans la formule YₓVO₄Eu₁₋ₓ.

La technique sol-gel est une technique bien connue de l'homme du métier et elle ne sera donc pas décrite de manière détaillée dans la présente.

On peut simplement rappeler que cette technique consiste en une polymérisation minérale en solution généralement réalisée dans des conditions douces de température et de pression.

Elle comprend une réaction d'hydrolyse d'un ou plusieurs précurseur(s) d'oxyde dans une phase solvant pour former des fonctions, groupements hydroxyles réactifs.

Ces fonctions, groupements hydroxyles réagissent ensuite par des réactions de condensation pour former des ponts "*oxo*" c'est-à-dire des ponts « métal-oxygène-métal » par élimination de molécules d'eau ou d'alcool.

Ainsi, par exemple, dans le cas de précurseurs organiques d'oxyde d'un métal ou métalloïde M, ces réactions s'écrivent schématiquement :

| | |
|---|---|
| Hydrolyse : | M-OR + H₂O → M-OH + R-OH |
| | |
| Condensation : | M-OH + RO-M → M-O-M + R-OH |
| | M-OH + HO-M → M-O-M + H₂O. |

Ces réactions d'hydrolyse-condensation conduisent tout d'abord à la formation d'une solution qui est appelée "*sol*" mais qui peut être, selon les conditions dans lesquelles est réalisée l'hydrolyse des précurseurs d'oxyde, soit un sol au sens strict du terme (c'est-à-dire une solution renfermant des espèces chimiques sous la forme de colloïdes), soit une solution renfermant des espèces chimiques sous forme d'oligomères ou de polymères, puis à la formation d'un *"gel"* (c'est-à-dire une masse visqueuse, élastique et présentant une structure de liquide figé) qui est constitué d'un réseau polymérique minéral et dont la viscosité augmente avec le temps.

Après élimination de la phase solvant emprisonnée dans le gel, ce dernier peut être soumis à des traitements complémentaires comme, par exemple, un traitement thermique permettant de le densifier.

Avantageusement, les nanoparticules notamment de YₓVO₄ : Eu₁₋ₓ peuvent avoir une taille, définie par leur plus grande dimension, par exemple par leur diamètre, de 2 à 100 nm, de préférence de 10 nm à 20 nm.

En effet, les nanoparticules mises en oeuvre selon l'invention ont généralement une forme sphérique ou quasi-sphérique, sphéroïdale.

Le sol, renferme typiquement des colloïdes, nanoparticules, d'oxyde d'au moins un métal de transition dopé par une terre rare, par exemple d'oxyde de vanadium et d'yttrium dopé par de l'europium présentant une taille, définie par leur plus grande dimension, par exemple par leur diamètre, de l'ordre de 2 nanomètres à 100 nanomètres, de préférence de 10 nm à 20 nm.

Ce sol, nommé sol colloïdal, est de préférence, préparé par hydrolyse basique des précurseurs d'oxydes en phase aqueuse.

Avantageusement, les précurseurs sont choisis parmi les sels métalliques tels que les nitrates.

Des exemples de précurseurs sont le nitrate d'europium, l'orthovanadate de sodium et le nitrate d'yttrium.

Afin de limiter la croissance des particules et d'assurer leur dispersion, une solution d'un dispersant tel que le citrate de sodium peut être ajoutée au mélange réactionnel.

L'élimination des ions en excès du sol peut être réalisée par dialyse en utilisant par exemple une membrane semi-perméable.

Lors de cette étape d'élimination des ions en excès, par exemple par dialyse, on peut également remplacer tout ou partie de l'eau par un solvant organique.

Ce solvant organique peut être choisi par exemple parmi les alcools aliphatiques de 1 à 6C tel que le méthanol, l'éthanol, et l'isopropanol ; l'acétonitrile ; le tétrahydrofuranne (THF) ; le toluène ; et leurs mélanges.

Quelle que soit la voie de synthèse choisie, à savoir hydrolyse basique de précurseur d'oxydes en phase aqueuse , ou autre, un sol stable, transparent et incolore est obtenu. La concentration du sol final est enfin ajustée par addition ou évaporation de solvant.

Le sol colloïdal d'oxydes obtenu comprend donc un solvant choisi généralement parmi l'eau ; les alcools aliphatiques de 1 à 6C tel que le méthanol, l'éthanol, et l'isopropanol ; l'acétonitrile ; le tétrahydrofuranne (THF) ; le toluène ; et leurs mélanges.

Un sol final qui comprend outre de l'eau un solvant organique, par exemple un sol final contenant un mélange d'eau et d'acétonitrile, de préférence en proportions égales, conserve la stabilité et toutes les propriétés du sol aqueux telles que les propriétés de fluorescence, mais présente l'avantage de permettre la solubilisation de peroxydes organiques comme le TATP.

Le sol colloïdal présente avantageusement une concentration en nanoparticules de 0,1% à 10% en masse, de préférence de 0,1% à 4% en masse, de préférence encore de 2% à 4% en masse.

Le dépôt de la couche ou des couches du sol d'oxydes colloïdaux sur le substrat peut être avantageusement réalisé par une technique de dépôt par voie humide.

Cette technique de dépôt par voie humide peut être choisie parmi l'une des techniques de dépôt par voie humide suivantes :
- la pulvérisation (connue sous la terminologie anglaise "*spray-coating*") ;
- l'enduction centrifuge (connue sous la terminologie anglaise "*spin-coating*") ;
- le dépôt à la goutte (connu sous la terminologie anglaise "*drop-coating*") ;
- le trempage-retrait (connu sous la terminologie anglaise "*dip-coating*") *;*
- l'enduction laminaire (connue sous la terminologie anglaise "*meniscus-coating*") ;
- l'épandage (connu sous la terminologie anglaise "*soak-coating*") ;
- le jet d'encre (connu sous la dénomination anglaise « *inkjet printing* ») ;
- l'enduction au rouleau (connue sous la terminologie anglaise "*roll to roll process*") ; ou encore
- l'enduction au pinceau (connue sous la terminologie anglaise "*paint-coating*").

Parmi ces techniques, qui sont bien connues de l'homme du métier, on préfère la pulvérisation, l'enduction centrifuge, le trempage-retrait et le dépôt à la goutte car ce sont celles qui conviennent le mieux à la réalisation d'un film mince par dépôt d'un sol d'oxydes colloïdaux sur un substrat.

Le trempage-retrait et l'enduction centrifuge sont encore préférés car ces techniques permettent d'obtenir des films minces homogènes.

Avantageusement, les nanoparticules d'oxyde d'au moins un métal de transition dopé par une terre rare, tel que YₓVO₄ : Eu₁₋ₓ, peuvent être chacune recouvertes, enrobées, d'une fine couche de silice obtenue par voie sol-gel.

Ces nanoparticules sont des nanoparticules à structure coeur-coquille (« *core shell* » en anglais) dont le coeur est constitué par une nanoparticule d'oxyde d'au moins un métal de transition dopé par une terre rare, tel que YₓVO₄ : Eu₁₋ₓ, et la coquille est constituée par une fine couche de silice.

Cette fine couche de silice présente généralement une épaisseur de 1 à 10 nanomètres.

Une telle épaisseur est généralement suffisante et permettra d'une part d'augmenter la stabilité des particules en milieu acide et d'autre part de greffer des groupements organiques susceptibles d'améliorer les performances du capteur chimique de gaz, par exemple sa sensibilité, sa sélectivité et sa résistance à l'humidité ambiante.

Cette fine couche de silice est de préférence obtenue par croissance en solution de silice sur la surface des nanoparticules d'oxyde d'au moins un métal de transition dopé par une terre rare comme l'europium, par exemple sur la surface des nanoparticules de YₓVO₄Eu₁₋ₓ.

Pour cela, on peut par exemple ajouter un précurseur de silice tel que le TEOS à un sol, par exemple un sol hydro-alcoolique contenant des nanoparticules d'oxyde d'au moins un métal de transition dopé par une terre rare comme l'europium, par exemple un sol contenant des nanoparticules de YₓVO₄Eu₁₋ₓ.

En présence d'un catalyseur qui peut être un acide ou une base, une fine couche de silice enrobe les nanoparticules d'oxyde d'au moins un métal de transition dopé par une terre rare comme l'europium, par exemple les nanoparticules de YₓVO₄Eu₁₋ₓ.

On obtient ainsi un sol de nanoparticules coeur-coquille (« core-shell » en anglais).

Ce sol, qui renferme typiquement des colloïdes ou nanoparticules d'oxyde d'au moins un métal de transition dopé par une terre rare comme l'europium, par exemple des colloïdes de YₓVO₄Eu₁₋ₓ recouverts de silice présentant un diamètre de l'ordre de 2 nanomètres à 100 nanomètres, de préférence de 10 à 20 nm. peut ainsi, de préférence, être préparé par hydrolyse basique d'au moins un précurseur d'oxyde de silicium dans une phase solvant contenant un solvant organique, seul ou mélangé à de l'eau, et les nanoparticules d'oxyde d'au moins un métal de transition dopé par une terre rare comme l'europium, par exemple les nanoparticules de YₓVO₄Eu₁₋ₓ, puis mûrissement de la solution ainsi obtenue.

Dans ce cas, la fine couche de silice est tout préférentiellement préparée par un procédé de croissance en solution de la silice qui comprend les étapes suivantes :
a) on prépare un sol de nanoparticules d'oxyde d'au moins un métal de transition dopé par une terre rare comme l'europium, par exemple des nanoparticules de YₓVO₄Eu₁₋ₓ dans un solvant constitué par un alcool ou un mélange d'alcools ;
b) on ajoute au sol préparé dans l'étape a) au moins un précurseur de silice, et au moins une base forte jouant le rôle de catalyseur, telle que l'ammoniaque, de façon à ce que le sol présente un pH au moins égal à 8;
c) on réalise l'hydrolyse dudit au moins un précurseur de silice dans l'alcool ou le mélange d'alcools additionné d'au moins une base forte, telle que l'ammoniaque, et présentant un pH au moins égal à 8, moyennant quoi il se forme une fine couche de silice sur les particules d'oxyde d'au moins un métal de transition dopé par une terre rare ;
d) on effectue le mûrissement de la solution ainsi obtenue pour former un sol alcoolique de nanoparticules d'oxyde revêtues d'une fine couche de silice, et éventuellement
e) on élimine la base forte du sol alcoolique ainsi formé ou on remplace par de l'eau tout ou partie de l'alcool ou des alcools présents dans ce sol.

Ainsi, le sol de nanoparticules d'oxyde d'au moins un métal de transition dopé par une terre rare comme l'europium, par exemple le sol de nanoparticules de YₓVO₄ : Eu₁₋ₓ recouvertes de silice peut aussi bien être un sol alcoolique, un sol hydro-alcoolique qu'un sol aqueux.

L'élimination de la base forte du sol obtenu à l'étape d) ci-dessus peut être réalisée en traitant ce dernier au reflux par un solvant organique, par exemple un alcool ou un mélange d'alcools.

Quant au remplacement par de l'eau de tout ou partie de l'alcool ou des alcools présents dans le sol obtenu à l'étape d) ci-avant, celui-ci peut être réalisé par dilution de ce sol par un mélange d'eau et d'un ou plusieurs alcool(s) pour obtenir un sol hydro-alcoolique, puis par concentration du sol hydro-alcoolique ainsi obtenu dans des conditions qui permettent d'éliminer de tout ou partie de l'alcool ou des alcools qu'il renferme.

Avantageusement, la fine couche de silice est préparée en milieu alcoolique avec une teneur contrôlée en eau et en ammoniaque.

En variante, le sol de particules d'oxyde d'au moins un métal de transition dopé par une terre rare comme l'europium, par exemple le sol de nanoparticules de YₓVO₄Eu₁₋ₓ peut être revêtu d'une fine couche de silice préparée par hydrolyse acide (c'est-à-dire que l'on utilise un acide en lieu et place de la base forte telle que l'ammoniaque) d'au moins un précurseur d'oxyde de silicium en solution dans une phase solvant contenant un solvant organique, seul ou mélangé à de l'eau, et dont le pH est, de préférence, compris entre 0 et 1, puis mélange de la solution ainsi obtenue avec une solution contenant de l'eau, un solvant organique, et mûrissement du mélange résultant.

Quelle que soit la voie d'hydrolyse retenue, le précurseur d'oxyde de silicium est, de préférence, choisi parmi les alcoxydes de silicium tétrafonctionnels de formule Si(OR)₄ (tétraalcoxysilanes) dans laquelle R représente un groupe alkyle comprenant de 1 à 6 atomes de carbone et, notamment, parmi le tétraméthylortho-silicate (TMOS) et le tétraéthylorthosilicate (TEOS).

Toutefois, d'autres types de précurseur d'oxyde sont également susceptibles d'être utilisés comme, par exemple :
- des alcoxydes tétrafonctionnels de formule Si(OR)₄ dans laquelle R est un groupe autre qu'un groupe alkyle, tel qu'un groupe acétyle ;
- des alcoxydes de silicium mono-, di- ou trifonctionnels de formule Si(OR)₄₋ₓR'ₓ dans laquelle R est un groupe alkyle en C₁-C₆, tandis que R' est un groupe organique ou inorganique, par exemple un atome de chlore, et x est égal à 1, 2 ou 3 ; ou encore
- des silicates inorganiques tels que SiCl₄, SiBr₄ ou Si₃O₇Na₂.

Le solvant organique est, lui, de préférence, un alcool ou un mélange d'alcools, en particulier un alcool aliphatique ou un mélange d'alcools aliphatiques comprenant de 1 à 6 atomes de carbone tels que le méthanol, l'éthanol ou l'isopropanol.

Toutefois, d'autres types de solvants organiques peuvent aussi être utilisés comme, par exemple, des phénols ou des diols de formule OH-R"-OH dans laquelle R" est un groupe alkylène en C₂-C₃₀ ou un groupe phényle.

Par ailleurs, le sol de nanoparticules d'oxyde d'au moins un métal de transition dopé par une terre rare, tel que YₓVO₄ : Eu₁₋ₓ, recouvertes de silice présente avantageusement une teneur, concentration massique en nanoparticules de 0,1% à 10%, de préférence, de 0,1% à 4%, et de préférence encore de 2% à 4% (la teneur est la même que pour le sol dont les nanoparticules ne sont pas recouvertes de silice).

Le dépôt de la couche ou des couches du sol sur des nanoparticules d'oxyde d'au moins un métal de transition dopé par une terre rare, mais cette fois revêtues de silice, sur le substrat, peut être effectué par une technique de dépôt par voie humide et notamment par l'une des techniques de dépôt par voie humide déjà citées plus haut.

Conformément à l'invention, et comme on l'a déjà indiqué plus haut, la silice peut être fonctionnalisée par des groupes chimiques par exemple des groupes chimiques susceptibles d'améliorer les performances du capteur chimique, par exemple en renforçant sa sensibilité et/ou sa sélectivité vis-à-vis de certains composés tels que les peroxydes, en augmentant sa réversibilité et/ou sa durabilité, ou en diminuant sa sensibilité aux variations hygrométriques environnementales, ou encore en améliorant sa robustesse, sa résistance à l'humidité ambiante.

Ainsi, la sensibilité et la sélectivité du capteur chimique vis-à-vis des composés peroxydes peuvent être renforcées par un greffage de groupes chimiques présentant une forte affinité pour ce type de composés, auquel cas ce greffage est, par exemple, réalisé en faisant réagir des groupes hydroxyles libres de la silice avec un composé de formule X_{(4-x-y-z)}-SiR¹ₓR²_{y}R³_{z} dans laquelle X est un groupe hydrolysable comme un halogénure, un acétonate, un acrylate ou un alcoolate de formule OR⁴ où R⁴ est un groupe alkyle comprenant de 1 à 10 atomes de carbone, R¹, R² et R³, qui peuvent être identiques ou différents, sont des groupes sensibles aux composés peroxydes, par exemple du type phtalocyanine de métal, x, y et z sont des nombres entiers allant de 0 à 3, à la condition toutefois que l'un au moins de x, y et z soit différent de 0 et que x+y+z ≤ 3.

La sensibilité du capteur chimique aux variations hygrométriques environnementales peut, elle, être diminuée par un greffage de groupes hydrophobes et, notamment, de groupes alkyles, de groupes aromatiques (phényles par exemple) ou de groupes fluorés comme, par exemple, des chaînes alkyles comportant plusieurs atomes de fluor du type -CH₂-CH₂-(CF₂)₇-CF₃, auquel cas ce greffage est, par exemple, réalisé en faisant réagir des groupes hydroxyles libres de la silice avec un composé de formule X_{(4-x-y-z)}-SiR¹ₓR²_{y}R³_{z} dans laquelle X, x, y et z ont la même signification que précédemment, mais dans laquelle R¹, R² et R³ sont des groupes hydrophobes.

Avantageusement, la fonctionnalisation, le greffage, de la silice peut se faire en faisant réagir les groupes hydroxyles libres de la silice avec un composé de type alcoxy (1 à 10C) silane présentant au moins un groupement non hydrolysable lié au silicium.

Quels que soient les groupes chimiques que l'on entend greffer sur la silice, le greffage de ces groupes peut être réalisé sur les nanoparticules d'au moins un oxyde d'au moins un métal de transition, tel que YₓVO₄:Eu₁₋ₓ, recouvertes de silice aussi bien lorsqu'elles sont à l'état de sol colloïdal, avant que celui-ci ne soit déposé sur le substrat, que sous forme de film mince solide (solvant éliminé), une fois celui-ci constitué sur le substrat.

Dans ce dernier cas, le greffage est effectué en mettant ce film mince en contact avec un composé comprenant au moins un groupe correspondant à ceux que l'on souhaite greffer, soit sous forme vapeur si les composés que l'on souhaite greffer sont volatils, soit sous forme liquide s'ils ne le sont pas.

Par exemple, on pourra tremper la fine couche de silice, constituée sur les nanoparticules dans une solution alcoolique contenant l'espèce à greffer et une base forte telle que de l'ammoniaque.

Par ailleurs, indépendamment de toute fonctionnalisation de la silice, la fine couche de silice sur les nanoparticules peut être soumise à un ou plusieurs traitement(s) choisi(s) parmi :
- un lavage par un solvant organique ;
- une densification par voie thermique ou radiative (UV, IR ou micro-ondes) ; ou encore
- un durcissement par voie chimique.

La densification par voie thermique, qui consiste à chauffer la fine couche de silice, à une température élevée, c'est-à-dire pouvant aller jusqu'à 900°C, mais toutefois inférieure à la température d'endommagement du substrat, permet de consolider cette fine couche.

Le durcissement par voie chimique, qui est décrit dans FR-A-2 703 791 [19], consiste à faire subir à la fine couche de silice, un traitement alcalin en milieu liquide ou gazeux, typiquement en présence de molécules d'ammoniac, ou un traitement acide. Il permet d'améliorer, non seulement la tenue mécanique de cette fine couche et, en particulier, sa résistance à l'abrasion et son adhésion sur le substrat, mais également les performances de détection du capteur chimique.

Selon la présente invention, les nanoparticules se présentent dans le capteur sous la forme d'un sol de nanoparticules d'au moins un oxyde d'au moins un métal de transition dopé par une terre rare, tel que YₓVO₄: Eu₁₋ₓ, obtenues par voie sol-gel, dans un solvant.

La présente invention a donc pour objet l'utilisation de nanoparticules d'au moins un oxyde d'au moins un métal de transition dopé par une terre rare, obtenues par un procédé sol-gel, en tant que matériau sensible dans un capteur chimique pour la détection ou le dosage de vapeurs d'un ou plusieurs composé(s) ou molécule(s) cible(s) ; dans laquelle les nanoparticules se présentent dans le capteur sous la forme d'un sol de nanoparticules dans un solvant.

Ce mode de réalisation selon l'invention où les nanoparticules se présentent dans le capteur sous la forme d'un sol de nanoparticules, fait intervenir une interaction liquide-gaz.

En d'autres termes, l'invention concerne également l'utilisation directe de sols généralement colloïdaux contenant des nanoparticules inorganiques d'au moins un oxyde d'au moins un métal de transition dopé par une terre rare, par exemple des nanoparticules de YₓVO₄ : Eu₁₋ₓ, obtenues par voie sol-gel en tant que matériau sensible dans un capteur chimique pour la détection ou le dosage de vapeurs d'un ou plusieurs composé(s) ou molécule(s) cible(s) tels que des peroxydes.

Un tel sol contenant des nanoparticules d'au moins un oxyde d'au moins un métal de transition dopé par une terre rare, par exemple des nanoparticules de YₓVO₄ : Eu₁₋ₓ, obtenues par voie sol-gel, ainsi que sa préparation, ont déjà été décrits plus haut.

Ce sol renferme typiquement des colloïdes d'au moins un oxyde d'au moins un métal de transition dopé par une terre rare telle que l'europium présentant une taille, définie par leur plus grande dimension telle que leur diamètre, de l'ordre de 2 nanomètres à 100 nanomètres.

Les nanoparticules d'oxydes de ce sol ont aussi déjà été décrites plus haut. Ces nanoparticules peuvent, comme on l'a déjà mentionné ci-dessus, être éventuellement pourvues d'une fine couche de silice et cette fine couche peut éventuellement être fonctionnalisée comme cela a déjà été précisé.

Ce sol colloïdal d'oxydes comprend un solvant choisi généralement parmi l'eau ; les solvants organiques tels que les alcools aliphatiques de 1 à 6C comme le méthanol, l'éthanol, et l'isopropanol, l'acétonitrile, le tétrahydrofuranne (THF), le toluène ; et leurs mélanges.

Le sol utilisé dans ce deuxième mode de réalisation a avantageusement une concentration en nanoparticules de 0,01% à 4% en masse.

Ce sol peut être utilisé directement. Il est alors mis en contact avec une atmosphère gazeuse, par exemple une atmosphère ou un volume d'air à analyser en vue de détecter ou de doser des vapeurs de composés ou molécules cibles, en particulier des vapeurs de polluants atmosphériques, tels que des vapeurs de peroxydes, susceptibles de se trouver dans cette atmosphère gazeuse.

Cette mise en contact sol-gaz peut se faire par exemple en projetant, nébulisant le sol, sous la forme d'un jet, nébulisât ou brouillard (« *spray* ») dans l'atmosphère gazeuse à analyser, ou en plaçant le sol dans une cuve et en mettant en contact, par exemple par barbotage, l'atmosphère gazeuse, le gaz avec le sol liquide.

Cette mise en contact est généralement couplée avec une détection optique utilisant la variation de l'intensité de fluorescence du matériau.

Ou bien, le sol de nanoparticules se présente sous la forme d'un film mince, par exemple d'un film mince recouvrant au moins en partie l'une ou les deux faces d'un substrat plan.

Il faut alors généralement prendre garde que le solvant ne s'évapore pas.

Ce substrat peut être par exemple un substrat de type microbalance à quartz pour un capteur gravimétrique ou bien une lame de quartz ou de verre pour un capteur optique.

Avantageusement, le film mince du sol de nanoparticules a une épaisseur de 2 nanomètres à 10 micromètres, de préférence de 2 nanomètres à un micromètre.

Le dépôt du film de sol peut être réalisé par l'une des techniques déjà mentionnées plus haut dans le cadre de la description de la préparation d'un film mince solide de nanoparticules. Il est toutefois important de noter que selon la présente invention, on n'élimine pas la phase solvant présente dans le film car celui-ci est un film du sol et non un film solide.

Ou bien encore, le sol peut être placé dans une cuve ou récipient avantageusement pourvu d'un couvercle amovible ce qui limite l'évaporation du solvant.

Conformément à l'invention, le substrat, ainsi que le système de mesure qui lui est associé, sont choisis en fonction de la propriété physique du film mince de sol, dont les variations induites par la présence d'un ou plusieurs composé(s) cible(s), notamment d'un ou plusieurs composé(s) de type peroxyde, sont destinées à être mesurées par le capteur chimique.

De préférence, on mesure les variations de masse et les variations de fluorescence de ce film mince.

Aussi, le capteur chimique est-il, de préférence, un capteur gravimétrique ou un capteur optique à fluorescence.

A titre d'exemples de capteurs gravimétriques, on peut citer les capteurs à microbalance à quartz, les capteurs à ondes de surface (connus sous la terminologie anglaise "SAW" pour "*Surface Acoustic Wave*") tels que les capteurs à ondes de Love et les capteurs à ondes de Lamb, et les microleviers.

Parmi les capteurs gravimétriques, on préfère plus particulièrement les capteurs du type microbalance à quartz.

Ce type de capteur, dont le principe de fonctionnement a été décrit par Sanchez-Pedrono et al. (Anal. Chem. Acta, 182, 1986, page 285, [21]), comprend, schématiquement, un substrat piézo-électrique (ou résonateur), généralement un cristal de quartz recouvert sur ses deux faces d'une couche métallique, par exemple d'or ou de platine, et qui est relié à deux électrodes. Le matériau sensible recouvrant l'une ou les deux faces du substrat, toute variation de masse de ce matériau se traduit par une variation de la fréquence de vibration du substrat.

Le principe de fonctionnement des capteurs optiques à fluorescence a, lui, été décrit par Valeur dans Molecular Fluorescence: Principles and Applications, 2002, Ed. WILEY VCH, New York [22]. Ce type de capteur comprend généralement un substrat en verre de qualité optique dont l'une des faces est recouverte d'un film mince du matériau sensible. L'intensité de la fluorescence émise par le matériau sensible peut être mesurée sur l'ensemble du spectre d'émission de ce matériau. Toutefois, il est préférable d'effectuer les mesures d'intensité de fluorescence à la longueur d'onde d'émission donnant les valeurs d'intensité maximales pour la longueur d'onde d'excitation conduisant, elle, au meilleur rapport signal/bruit pour l'acquisition des intensités de fluorescence.

Le capteur chimique peut également être un multicapteur, c'est-à-dire qu'il peut comprendre plusieurs capteurs élémentaires qui sont munis de matériaux sensibles, de substrats et/ou de systèmes de mesure différents les uns des autres.

Le multicapteur pourra comprendre par exemple plusieurs capteurs élémentaires choisis parmi les capteurs gravimétriques et les capteurs optiques à fluorescence, l'essentiel étant que l'un au moins parmi ces capteurs élémentaires soit un capteur qui comprend des nanoparticules d'au moins un oxyde d'au moins un métal de transition dopé par une terre rare obtenues par un procédé sol-gel en tant que matériau sensible ; ces nanoparticules pouvant se présenter sous la forme d'un film mince de nanoparticules, par exemple de YₓVO₄:Eu₁₋ₓ tel que précédemment décrit, ou d'un sol de nanoparticules, par exemple de YₓVO₄:Eu₁₋ₓ·

Conformément à l'invention, le ou les composé(s) ou molécule(s) cible(s) destiné(s) à être détecté(s) par le capteur chimique est (sont) choisi(s) de préférence parmi les polluants atmosphériques tels que H₂O₂, les explosifs et les précurseurs d'explosifs.

De préférence encore, le ou les composé(s) ou molécule(s) cible(s) destiné(s) à être détecté(s) par le capteur chimique est (sont) chois(s) parmi les peroxydes.

Avantageusement, selon l'invention, on réalise la détection ou le dosage sélectif des peroxydes vis-à-vis des composés organiques volatils.

Conformément à l'invention, les peroxydes destinés à être détectés par le capteur sont, de préférence, des peroxydes dont au moins l'un des deux atomes d'oxygène du groupe ou de l'un des groupes -O-O- qu'ils comportent est lié à un atome d'hydrogène, moyennant quoi cet atome d'oxygène forme un groupe hydroxyle avec cet atome d'hydrogène.

Des peroxydes qui répondent à ce critère sont notamment le peroxyde d'hydrogène, les hydro-peroxydes comme, par exemple, l'hydroperoxyde de t-butyle, l'hydroperoxyde d'α-cumyle ou et l'hydro-peroxyde de 1-phénéthyle, les peroxydes de cétones comme, par exemple, le peroxyde de méthyléthylcétone, le peroxyde d'acétylacétone, le peroxyde de cyclohexanone, le peroxyde d'acétone (diperoxyde d'acétone C₆H₁₂O₄, triperoxyde d'acétone -ou triperoxyde de triacétone- C₉H₁₈O₆ et tétraperoxyde d'acétone C₁₂H₂₄O₈), et le triperoxyde d'hexaméthylène diamine, le peroxyde d'hydrogène étant particulièrement préféré.

L'utilisation de nanoparticules de YₓVO₄:Eu₁₋ₓ, obtenues par un procédé sol-gel en tant que matériau sensible dans un capteur chimique pour la détection ou le dosage de vapeurs d'un ou plusieurs composé(s) ou molécule(s) cible(s) tels que des peroxydes s'est révélée présenter de nombreux avantages qui ont été mis en évidence lors d'essais réalisés avec ce matériau qui sont notamment exposés dans les exemples qui suivent.

Ces avantages sont notamment les suivants :
- les capteurs munis d'un tel matériau sensible sont capables de détecter spécifiquement des vapeurs de peroxydes, et en particulier des vapeurs de peroxydes d'hydrogène, avec une très grande sensibilité puisqu'ils peuvent détecter leur présence à des concentrations dans l'air de l'ordre de la ppm (partie par million) et même inférieures;
- les capteurs munis de ce matériau sensible sont caractérisés en particulier par :
   - une rapidité de réponse et une reproductibilité de cette réponse ;
   - une sélectivité vis-à-vis des composés peroxydés;
   - une stabilité de leurs performances dans le temps et une durée de vie très satisfaisante ;
   - une stabilité de leurs performances dans une large gamme d'hygrométrie ambiante ;
   - une aptitude à fonctionner en continu ;
   - une simplicité de fabrication liée au fait que le procédé sol-gel est un procédé simple à mettre en oeuvre ;
   - un coût compatible avec une production des capteurs en série ; et
   - la possibilité d'être miniaturisés et, ainsi, d'être aisément transportables et manipulables sur tout type de site.
- le matériau est constitué de particules de taille nanométrique, ce qui confère au sol contenant ces nanoparticules pulvérisé en gouttelettes, une grande surface d'échange avec l'atmosphère gazeuse ;
- l'interaction a lieu directement entre le composé cible et le matériau sensible dès la température ambiante ;

La détection peut notamment être caractérisée par une variation de l'intensité de fluorescence du matériau.
- la nature inorganique du matériau sensible, constitué d'oxydes métalliques, lui confère une robustesse très supérieure à celle des matériaux organiques, en particulier une grande stabilité des performances dans le temps et sous irradiation, notamment à la lumière du jour, avec par exemple, une intensité de fluorescence conservée pour des durées de vie supérieures à trois mois, que le matériau soit sous la forme d'un sol ou d'un film mince solide ;
- le matériau sensible en particulier lorsqu'il s'agit de composés du type YₓVO₄:Eu₁₋ₓ possède avantageusement des propriétés de fluorescence lorsqu'il est soumis à un rayonnement ultraviolet. Cette caractéristique permet une utilisation de ce matériau dans un capteur optique pour la détection de peroxydes, et en particulier des vapeurs de peroxyde d'hydrogène, par extinction de la fluorescence ;

En effet, ces composés se caractérisent par une diminution extrêmement rapide de leur intensité de fluorescence en présence de peroxydes sous forme de vapeurs.

La diminution de l'intensité de fluorescence en présence de peroxyde est due à l'interaction hétérogène entre les vapeurs de l'analyte tel que le peroxyde et le matériau sensible inhibant le transfert d'énergie depuis l'oxyde de métal de transition tel qu'un vanadate vers la terre rare telle que l'europium responsable de la fluorescence.

Un tel capteur est alors très sensible, sélectif vis-à-vis notamment des composés organiques volatils (comme les alcools, les cétones, les composés aromatiques cycliques ou les hydrocarbures), et fournit une réponse rapide, fiable, et reproductible.

Précisons que par « sélectif vis-à-vis des composés organiques volatils », on entend généralement que le capteur permet de détecter sélectivement les peroxydes dans un gaz comprenant aussi des composés organiques volatils. Cette sélectivité est montrée dans l'exemple 7, plus bas.

Enfin, l'émission de fluorescence de l'europium vers 617 nm fournit une réponse visuelle qui favorise l'interprétation de la détection.

L'invention concerne, en outre, un capteur chimique qui comprend des nanoparticules d'au moins un oxyde d'au moins un métal de transition dopé par une terre rare, obtenues par un procédé sol-gel, en tant que matériau sensible.

Dans ce capteur chimique, qui comprend des nanoparticules d'au moins un oxyde d'au moins un métal de transition dopé par une terre rare, obtenues par un procédé sol-gel, en tant que matériau sensible ; les nanoparticules se présentent dans le capteur sous la forme d'un sol de nanoparticules dans un solvant.

Ledit capteur peut être un capteur gravimétrique, de préférence un capteur à microbalance à quartz ou un capteur à fluorescence.

Le capteur est de préférence destiné à détecter le peroxyde d'hydrogène.

Les capteurs selon l'invention trouvent leur application pour la détection d'explosifs primaires ou artisanaux ou de précurseurs d'explosifs, que ce soit en vue d'assurer la sécurité des lieux publics (aéroports, métros, gares), de contrôler la licéité des marchandises en circulation sur un territoire, de lutter contre le terrorisme ou encore de dépolluer des sites industriels. Ils sont également utiles pour la protection de l'environnement, en particulier pour le contrôle et la surveillance de la pollution atmosphérique et de la qualité d'ambiances plus ou moins confinées, ainsi que pour la surveillance à des fins sécuritaires, de sites industriels fabriquant stockant ou manipulant des composés peroxyde.

D'autres caractéristiques et avantages de l'invention apparaîtront mieux à la lecture du complément de description qui suit, qui se rapporte à des exemples illustrant la préparation de sols de nanoparticules de EuVO₄ et de Y_{0,5}VO₄ : Eu_{0,5}, la préparation d'un film mince de nanoparticules de EuVO₄, l'utilisation pour la détection de vapeurs de peroxyde d'hydrogène de capteurs à fluorescence comprenant ce film mince, et la démonstration de la sélectivité, de la sensibilité et de la stabilité dans le temps de ces capteurs.

Bien entendu, les exemples qui suivent ne sont donnés qu'à titre d'illustrations de l'objet de l'invention et ne constituent en aucun cas une limitation de cet objet.

### BRÈVE DESCRIPTION DES DESSINS

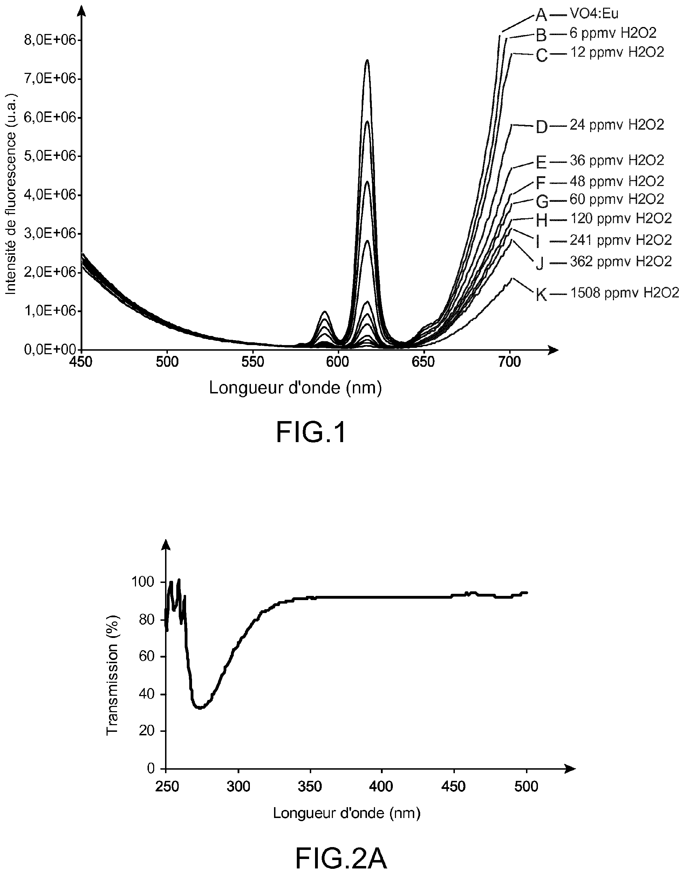

- La Figure 1 est un graphique qui montre des spectres d'émission de fluorescence, réalisés dans l'exemple 5, représentant l'évolution de l'émission de fluorescence en fonction de la longueur d'onde telle qu'elle est observée pour des solutions colloïdales de nanoparticules de EuVO₄ ne contenant pas de peroxyde d'hydrogène (courbe A) ou des quantités variables de H₂O₂ de 6, 12, 24, 36, 48, 60, 120, 241, 362, 1508 ppmv (courbes B, C, D, E, F, G, H, I, J, K).
   En abscisse est portée la longueur d'onde d'émission des solutions (en nm), et en ordonnée est portée l'intensité de fluorescence (en unités arbitraires).

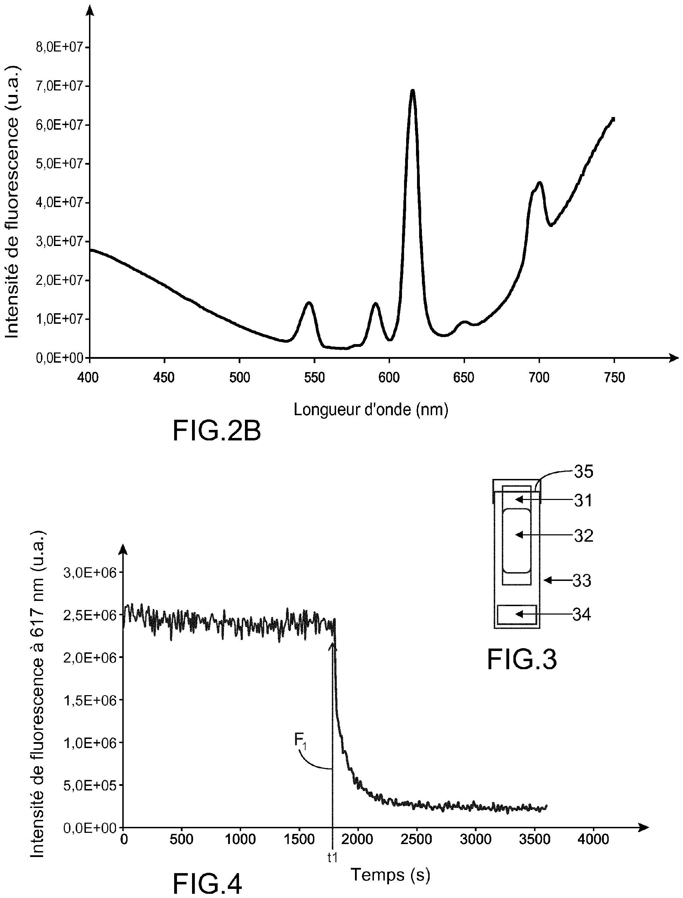

- La figure 2 représente les spectres d'absorption (Figure 2A) et d'émission (Figure 2B) d'un film mince solide constitué de nanoparticules de EuVO₄.
   En abscisse est portée la longueur d'onde d'absorption (Figure 2A) ou d'émission (Figure 2B) du film mince (en nm), et en ordonnée est portée la transmission (en %) ou l'intensité de fluorescence (en unités arbitraires) (Exemple 6).
- La figure 3 représente une vue schématique en coupe verticale du dispositif utilisé pour les tests de détection de vapeurs de H₂O₂. Ce montage permet de soumettre le capteur optique à une atmosphère saturée en peroxyde.
- La figure 4 représente l'évolution de l'intensité de fluorescence à 617 nm, après excitation à 270 nm, telle qu'observée pour un capteur optique comprenant un film mince solide constitué de nanoparticules de EuVO₄, lorsque ce capteur est successivement exposé à l'air ambiant puis à des vapeurs de peroxyde d'hydrogène.
   En abscisse est porté le temps (en secondes), et en ordonnée est portée l'intensité de fluorescence à 617 nm (en unités arbitraires).

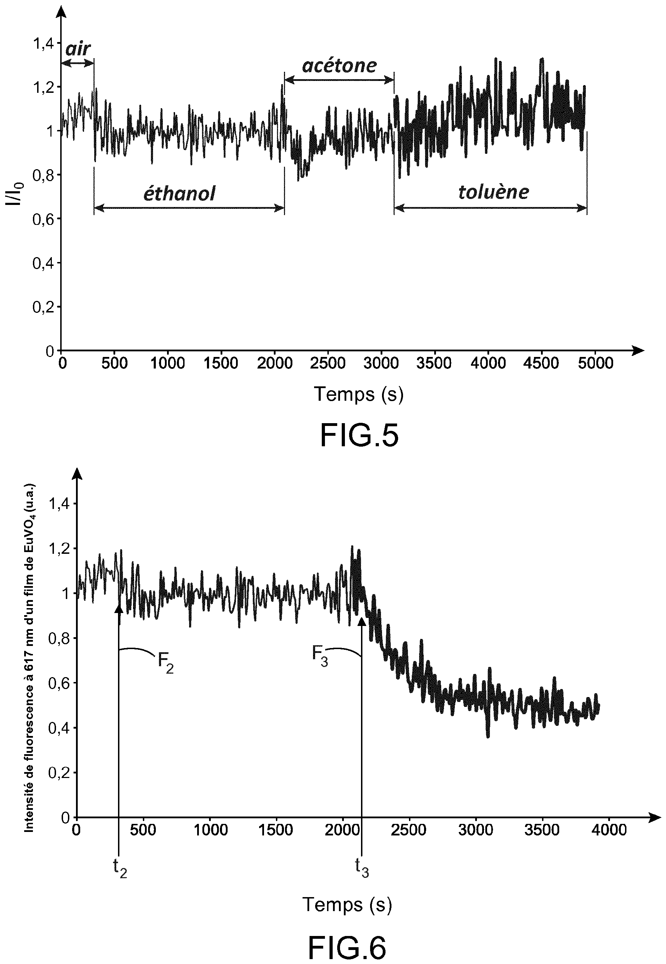

- La figure 5 représente l'évolution de l'intensité de fluorescence à 617 nm, après excitation à 270 nm, telle qu'observée pour un capteur optique comprenant un film mince solide constitué de nanoparticules de EuVO₄ lorsque ce capteur est exposé à l'air ambiant ou à des vapeurs de composés organiques volatils, à savoir des vapeurs d'éthanol, d'acétone, et de toluène.
- La figure 6 représente l'évolution de l'intensité de fluorescence à 617 nm, après excitation à 270 nm, telle qu'observée pour un capteur optique comprenant un film mince constitué de nanoparticules de EuVO₄ lorsque ce capteur est successivement exposé à l'air ambiant, à des vapeurs d'éthanol puis à des vapeurs de peroxyde d'hydrogène.

En abscisse est porté le temps (en secondes), et en ordonnée est portée l'intensité de fluorescence à 617 nm (en unités arbitraires).

### EXPOSÉ DÉTAILLÉ DE MODES DE RÉALISATION PARTICULIERS

### Exemples :

### Exemple 1.

Dans cet exemple, on prépare un sol aqueux de nanoparticules de EuVO₄ (x = 0 dans la formule indiquée plus haut).

On prépare tout d'abord des solutions aqueuses contenant les précurseurs d'oxydes, à savoir d'une part une solution de nitrate d'europium et d'autre part une solution d'orthovanadate de sodium, puis ces solutions sont mélangées à 60°C.

La solution de nitrate d'europium, dont la concentration est de 0,1 M, est préparée par dissolution de 1,71 g d'Eu(NO₃)₃, 5 H₂O dans 40 mL d'eau.

La solution d'orthovanadate de sodium, dont la concentration est de 0,1 M, est préparée à partir de 0,55 g de Na₃VO₄ qui sont versés dans 30 mL d'eau. Le pH de la solution de Na₃VO₄ est contrôlé et ajusté si nécessaire à une valeur comprise entre 12,3 et 12,8 par addition d'hydroxyde de sodium.

La solution de nitrate d'europium est introduite dans un ballon Bicol surmonté d'un réfrigérant et d'une ampoule d'addition.

La solution d'orthovanadate de sodium est placée dans cette ampoule d'addition et ajoutée goutte à goutte au contenu du ballon Bicol, sous agitation magnétique. Après addition de toute la solution d'orthovanadate, le sol obtenu est laissé sous agitation pendant 30 minutes à 60°C, durant lesquelles se déroule la réaction entre le nitrate d'europium et l'orthovanadate de sodium.

Après ce temps de réaction, le sol obtenu est ramené à température ambiante puis il est dilué 20 fois.

Ensuite, ce sol dilué est laissé 30 minutes dans un bain à ultrasons afin d'assurer une bonne dispersion des particules en phase aqueuse.

Puis le sol dilué subit une opération de dialyse contre de l'eau désionisée afin d'éliminer les sels.

L'eau est renouvelée deux fois par jour pendant trois jours.

On obtient finalement un sol aqueux de nanoparticules de EuVO₄ à pH 6 environ avec une teneur massique en inorganique (c'est-à-dire en EuVO₄) de 0,4% environ, qui peut éventuellement être filtré avant son utilisation.

### Exemple 2.

Dans cet exemple, on prépare un sol aqueux de nanoparticules de Y_{0,5}VO₄Eu_{0,5} en présence de citrate de sodium.

On prépare tout d'abord des solutions aqueuses contenant les précurseurs d'oxydes, à savoir une solution de nitrate d'europium, une solution d'orthovanadate de sodium, et une solution de nitrate d'yttrium, puis ces solutions de précurseurs sont mélangées avec une solution de citrate de sodium à 60°C.

La solution de nitrate d'europium, dont la concentration est de 0,1 M, est préparée par dissolution de 0,86 g d'Eu(NO₃)₃, 5 H₂O dans 20 mL d'eau.

La solution d'orthovanadate de sodium, dont la concentration est de 0,1 M, est préparée à partir de 0,55 g de Na₃VO₄ qui sont versés dans 30 mL d'eau. Le pH de la solution de Na₃VO₄ est contrôlé et ajusté si nécessaire à une valeur comprise entre 12,3 et 12,8 par addition d'hydroxyde de sodium.

La solution de nitrate d'yttrium, dont la concentration est de 0,1 M, est préparée à partir de 0,77 g de Y(NO₃)₃, 6 H₂O et de 20 mL d'eau.

La solution de citrate de sodium, dont la concentration est de 0,1 M, est obtenue par dissolution de 0,88 g de C₆H₅Na₃O₇, 2 H₂O dans 30 mL d'eau.

Les solutions de nitrate d'europium et de nitrate d'yttrium sont mélangées et introduites dans un ballon bicol surmonté d'un réfrigérant et d'une ampoule d'addition.

La solution de citrate de sodium est placée dans cette ampoule d'addition et est ensuite ajoutée goutte à goutte au contenu du ballon Bicol, sous agitation magnétique.

Un précipité blanc de citrate de lanthanide se forme alors.

La solution d'orthovanadate de sodium est placée dans l'ampoule d'addition.

Le précipité blanc de citrate de lanthanide est ensuite complètement dissous par addition goutte à goutte de la solution d'orthovanadate de sodium au contenu du Bicol sous agitation magnétique.

Après l'addition complète des différents réactifs, le sol est laissé sous agitation à 60°C pendant 30 minutes durant lesquelles se déroule la réaction entre le nitrate d'europium, l'orthovanadate de sodium et le nitrate d'yttrium.

Après ce temps de réaction, le sol obtenu est ramené à la température ambiante puis il subit une opération de dialyse contre de l'eau désionisée pendant trois jours, en renouvelant l'eau deux fois par jour afin d'éliminer les sels et l'excès de citrate de sodium.

On obtient alors un sol aqueux stable, transparent et incolore de nanoparticules de Y_{0,5}VO₄ : Eu_{0,5} à pH 7,5 environ avec une teneur massique en inorganique (Y_{0,5}VO₄Eu_{0,5}) de 0,5% environ.

### Exemple 3.

Dans cet exemple, on prépare un sol hydro-organique de nanoparticules de Y_{0,5}VO₄Eu_{0,5}.

Le sol aqueux de nanoparticules de Y_{0,5}VO₄Eu_{0,5} de l'exemple 2 est placé dans une membrane semi-perméable puis il subit une opération de dialyse contre une solution contenant un mélange d'eau et d'acétonitrile en proportions égales.

Le sol final conserve la stabilité ainsi que les propriétés de fluorescence du sol aqueux mais permet la solubilisation de peroxydes organiques comme le TATP par exemple.

### Exemple 4.

Dans cet exemple, on prépare un film mince constitué de nanoparticules de EuVO₄.

Afin de réaliser un film mince de EuVO₄ à partir du sol de l'exemple 1, il est nécessaire d'effectuer un transfert des particules dans un solvant organique.

Le solvant usuellement utilisé pour la mise en oeuvre de couches minces est l'éthanol, ou un mélange éthanol-eau.

Le sol de l'exemple 1 subit donc une opération de dialyse contre un mélange composé à 90% d'éthanol absolu et à 10% d'eau désionisée pendant 24 heures, moyennant quoi on obtient un sol hydro-alcoolique.

Les deux faces d'un substrat constitué par une lame de verre, de type lame de microscope, sont revêtues par trempage retrait du substrat dans le sol hydro-alcoolique obtenu ci-dessus à une vitesse de 15 cm.min⁻¹ ou par enduction centrifuge à une vitesse de 800 tr.min⁻¹.

On réalise un séchage pendant 5 minutes à l'air libre afin d'éliminer les solvants de la couche formée.

### Exemple 5.

Dans cet exemple, on étudie l'effet qu'ont des solutions de H₂O₂ présentant diverses concentrations sur le spectre d'émission d'une solution colloïdale de EuVO₄.

Pour observer l'effet d'une solution de H₂O₂ sur le spectre d'émission d'une solution colloïdale de EuVO₄, des cuves en quartz contenant un volume de liquide constant de 2,9 mL sont préparées.

Elles contiennent 1,5 mL de solution colloïdale, une quantité variable de peroxyde d'hydrogène à savoir 6, 12, 24, 36, 48, 60,120, 241, 362, et 1508 ppmv de H₂O₂, et sont complétées par de l'eau.

Une cuve contient seulement 1,5 mL de solution colloïdale sans peroxyde d'hydrogène.

Spectres d'émission des solutions : ils sont acquis par un spectrofluorimètre Horiba Jobin Yvon Fluoromax-P®.

La longueur d'onde d'excitation utilisée est de 270 nm. L'ouverture des fentes d'entrée des monochromateurs est ajustable (de 1 à 5 nm). Elle est choisie en fonction de l'intensité d'émission des solutions afin d'obtenir un signal suffisant, sans saturer le détecteur. Les cuves en quartz sont positionnées dans le porte échantillon du spectrofluorimètre.

Les spectres d'émission obtenus sont présentés sur la Figure 1.

La figure 1 met clairement en évidence l'affinité entre le matériau sensible et l'eau oxygénée en solution.

### Exemple 6.

Dans cet exemple, on réalise les spectres d'absorption (Figure 2A) et d'émission (Figure 2B) d'un film mince solide constitué de nanoparticules de EuVO₄ tel que celui préparé dans l'exemple 4.

Les protocoles d'acquisition des spectres d'absorption et d'émission sont présentés ci-dessous.

Spectres d'absorption des films : ils sont acquis par un spectromètre Perkin Elmer Lambda 900®.

Un balayage standard est utilisé entre 800 et 200 nm en transmission, avec un intervalle entre les données de 1 nm. Le blanc est fait à l'air.

Spectres d'émission des films : ils sont acquis par un spectrofluorimètre Horiba Jobin Yvon Fluoromax-P®.

La longueur d'onde d'excitation utilisée est de 270 nm. L'ouverture des fentes d'entrée des monochromateurs est ajustable (de 1 à 5 nm). Elle est choisie en fonction de l'intensité d'émission des films afin d'obtenir un signal suffisant, sans saturer le détecteur. Le substrat en quartz sur lequel est déposé le film est positionné dans la chambre de mesure avec un angle d'inclinaison de 45° par rapport au faisceau incident d'excitation.

Les spectres d'absorption et d'émission sont présentés respectivement sur les figures 2A et 2B.

Il est à noter que ces spectres concernent EuVO₄ mais que les spectres sont similaires quelle que soit la teneur en Eu.

### Exemple 7.

Dans cet exemple, on effectue la détection de vapeurs de peroxyde d'hydrogène à l'aide d'un capteur optique comprenant un film mince constitué de nanoparticules de EuVO₄.

Un spectrofluorimètre est utilisé en mode « cinétique », qui permet de mesurer l'intensité de fluorescence à une longueur d'onde donnée, dite longueur d'onde de mesure, sous une longueur d'onde d'excitation donnée, en fonction du temps.

La longueur d'onde d'excitation utilisée est 270 nm alors que la longueur d'onde de mesure est 617 nm.

Comme cela est représenté sur la Figure 3, un substrat de verre (31), sur lequel un film de EuVO₄ (32) a été déposé préalablement comme cela a été décrit dans l'exemple 4, est introduit dans une cuve en quartz (33), qui est elle-même positionnée dans le porte-échantillon du spectrofluorimètre (Horiba Jobin Yvon FluoroMax®).

Toutes les mesures sont effectuées à température ambiante et suivant le protocole décrit précédemment.

On démarre la mesure de cinétique afin de vérifier la stabilité de l'intensité de fluorescence du film en l'absence de vapeurs d'eau oxygénée.

Après 30 minutes d'exposition aux UV, la cuve en quartz est saturée en vapeurs de H₂O₂ en versant 0,5 mL d'une solution aqueuse de H₂O₂ à 17,5% en volume dans le fond de celle-ci, sans que la solution de H₂O₂ (34) n'entre en contact avec le film comme cela est illustré sur la Figure 3.

La cuve est ensuite rapidement fermée à l'aide de Parafilm® (35) afin de poursuivre la mesure de cinétique.

La diminution de la fluorescence à 617 nm due à l'interaction entre le film de EuVO₄ et les vapeurs de H₂O₂ est alors mesurée.

La Figure 4 représente l'intensité de fluorescence en absence, puis en présence de vapeurs d'eau oxygénée. Pendant les 1800 premières secondes, le film de EuVO₄ n'est pas exposé à H₂O₂ afin de vérifier la stabilité de l'intensité de fluorescence du film.

Après 1800 secondes (temps t1 sur la Figure 4), la solution d'eau oxygénée est introduite dans la cuve (flèche F1 sur la Figure 4) qui est aussitôt refermée. L'intensité de fluorescence diminue alors considérablement.

### Exemple 8.

Dans cet exemple, on met en évidence la sélectivité d'un capteur optique comprenant un film mince constitué de nanoparticules de EuVO₄ vis-à-vis des peroxydes.

Un spectrofluorimètre est utilisé en mode « cinétique », qui permet de mesurer l'intensité de fluorescence à une longueur d'onde donnée, dite longueur d'onde de mesure, sous une longueur d'onde d'excitation donnée, en fonction du temps.

La longueur d'onde d'excitation utilisée est 270 nm alors que la longueur d'onde de mesure est 617 nm.

Un substrat de verre, sur lequel un film de EuVO₄ a été déposé préalablement comme cela a été décrit dans l'exemple 4, est introduit dans une cuve en quartz, qui est elle-même positionnée dans le porte-échantillon du spectrofluorimètre (Horiba Jobin Yvon FluoroMax®).

Toutes les mesures sont effectuées à température ambiante et suivant le protocole décrit précédemment.

On démarre la mesure de cinétique afin de vérifier la stabilité de l'intensité de fluorescence du film.

Après 10 minutes, la cuve en quartz est saturée en vapeurs d'un solvant organique en versant 0,5 mL du solvant dans le fond de la cuve en quartz sans que le solvant n'entre en contact avec le film comme dans l'exemple 7.

La cuve est ensuite rapidement fermée à l'aide de Parafilm® et la mesure de cinétique est poursuivie. L'évolution de la fluorescence à 617 nm du film de EuVO₄ est donc mesurée en présence d'une atmosphère saturée par les vapeurs du solvant.

On effectue trois essais, chaque fois avec un solvant différent, à savoir l'éthanol, l'acétone, puis le toluène.

Soient I₀ l'intensité de fluorescence à 617 nm du film de EuVO₄ au temps to, et I l'intensité de fluorescence du même film à 617 nm au temps t.

La Figure 5 représente l'évolution de I/I₀ en fonction du temps en secondes, en présence d'un solvant (éthanol, acétone ou toluène) dans le fond de la cuve ou lorsque le capteur est simplement exposé à l'air ambiant avant addition de tout interférent.

Les mesures en présence des différents solvants et à l'air ambiant ont été effectuées séparément mais sont reportées sur le même graphique pour faciliter la comparaison.

Le rapport I/I₀ reste globalement constant. Le capteur est donc sélectif vis-à-vis des peroxydes et les solvants ne créent aucune interférence.

### Exemple 9.

Dans cet exemple, un substrat de verre, sur lequel un film de EuVO₄ a été déposé préalablement comme cela a été décrit dans l'exemple 4, est introduit dans une cuve en quartz, qui est elle-même positionnée dans le porte-échantillon du spectrofluorimètre (Horiba Jobin Yvon FluoroMax®).

Toutes les mesures sont effectuées à température ambiante et suivant le protocole décrit précédemment.

Le film est tout d'abord exposé à l'air ambiant, jusqu'à un temps t2 (300 s) auquel de l'éthanol est introduit dans la cuve (flèche F2 de la Figure 6).

Après l'exposition du capteur à des vapeurs d'éthanol pendant 1800 secondes, quelques gouttes de peroxyde d'hydrogène sont introduites (flèche F3 de la Figure 6) dans la cuve au temps t3 (2100 s), toujours sans que le film n'entre en contact avec le liquide.

La mesure de l'intensité de fluorescence du film de EuVO₄ est poursuivie et ses variations sont reportées sur la Figure 6.

Cette figure représente donc l'évolution du rapport I/I₀ en fonction du temps lorsque le film est placé dans la cuve vide pendant 300 secondes, en présence d'éthanol pendant 1800 secondes, puis en présence de peroxyde d'hydrogène pendant 2100 secondes.

La Figure 6 montre que l'intensité de fluorescence diminue après l'exposition au peroxyde d'hydrogène. Ceci indique que le capteur offre une bonne sensibilité et une très grande spécificité à l'égard des peroxydes malgré la présence de solvants organiques.

### Exemple 10.

Dans cet exemple, on met en évidence de la stabilité dans le temps des performances d'un capteur optique comprenant un film mince constitué de nanoparticules de EuVO₄.

Dans cet exemple, on utilise un capteur identique à celui des exemples 4 et 7 à 9.

Le jour de sa réalisation, le capteur est exposé à des vapeurs de peroxyde d'hydrogène, en atmosphère saturée dans la cuve de mesure, pendant 10 minutes.

La valeur de l'intensité de fluorescence est alors notée I₀. Ce capteur est ensuite stocké pendant 100 jours à l'air ambiant et à la lumière du jour. Une nouvelle mesure est alors effectuée sous exposition de vapeurs saturées de peroxyde d'hydrogène pendant 5 minutes. L'intensité de fluorescence est alors mesurée.

Les valeurs I₀ et I₁₀₀ étant identiques, de l'ordre de (1,3 ± 0,1).10⁷, on peut en conclure que les performances de fluorescence du capteur sont stables sur de longues périodes de temps, supérieures à 3 mois ici, et sans prendre de précautions particulières quant aux conditions de conservation de ce capteur contrairement aux matériaux organiques usuels. L'intensité de fluorescence à 617 nm d'un film de EuVO₄ excité à 270 nm est donc conservée malgré un stockage à l'air et à la lumière du jour. Le matériau a donc une durée de vie supérieure à trois mois sous exposition à la lumière du jour.

### RÉFÉRENCES

[1] S. Yao, S. Yuang, J, Xu, Y. Wang, J. Luo, S. Hu, Applied Clay Science, 33, 35, 2006
[2]
   (a) J.G. Hong, J. Maguhn, D. Freitag, A. Kettrup, Fresenius J. Anal. Chem., 361, 124, 1998
   (b) R. Schulte-Ladbeck, P. Kolla, U. Karst, Analyst, 127, 1352, 2002
[3] D. Lu, A. Cagan, R Minoz, T. Tangkuaram, J. Wang, Analyst, 131, 1279, 2006
[4] F. Bohrer, C. Colesniuc, J. Park, I. Schuller, A. Kummel, W. Trogler, Journal of American Chemical Society, 130,3712, 2008
[5] C. Guzman, G. Orozco, Y. Verde, S. Jimenez, L.A. Godinez, E. Juaristi, E. Bustos, Electrochimica Acta, 54, 1728, 2009
[6] D. Laine, C. Roske, F. Cheng, Anal. Chim. Acta, 608, 56, 2008
[7] R. Munoz, D. Lu, A. Cagan, J. Wang, Analyst, 132,560, 2007
[8] A. Mills, P. Grosshans, E. Snadden, Sensors and Actuators B, 136, 458, 2009
[9] A. Apblett, B. Kiran., S. Malka, N. Materer, A. Piquette, Ceram Trans, 172, 29, 2005
[10] J. Sanchez, W. Trogler, Journal of Material Chemistry, 18, 5134, 2008
[11] M. Germain, M. Knapp, Inorganic Chemistry, 47, 9748, 2008
[12] S. Malashikhin, N. Finney, Journal ofthe American Society, 130, 12846, 2008
[13] X. Shu, Y. Chen, H. Yuang, S. Gao, D. Xiao, Anal. Chem., 79(10), 3695, 2007
[14] D. Casanova, C. Bouzigues, T.L. Nguyên, R.O. Ramodiharilafy, L. Bouzhir-Sima, T. Gacoin., J.P. Boilot, P.L. Tharaux, A. Alexandrou, Nature Nanotechnology, 4, 581, 2009
[15] P. Belleville, C R. Chimie, 13,97, 2010
[16] H. Althues, P. Simon, S. Kaskel, Journal ofMaterial Chemistry, 17, 758, 2007
[17] M. Yu, .J. Lin, F. Fang, Chemistry of Materials, 17, 1783, 2005
[18] Q. Zhou, L. Zhang, H. Fan, L. Wu, Y. Lv, Sensors and Actuators B, 144, 192, 2010
[19] FR-A-2 703 791
[20] Rouquérol et al., Pure & Applied Chemistry, 66(8), 1994, pages 1739-1758
[21] Sanchez-Pedrono, Anal. Chem. Acta, 182, 285, 1986
[22] B. Valeur, Molecular Fluorescence: Principles and Applications, Ed. WILEY VCH, New York, 2002.

## Revendications

1. Utilisation de nanoparticules d'au moins un oxyde d'au moins un métal de transition dopé par une terre rare, obtenues par un procédé sol-gel, en tant que matériau sensible dans un capteur chimique pour la détection ou le dosage de vapeurs d'un ou plusieurs composé(s) ou molécule(s) cible(s) ; dans laquelle les nanoparticules se présentent dans le capteur sous la forme d'un sol de nanoparticules dans un solvant.

2. Utilisation selon la revendication 1, dans laquelle le sol de nanoparticules présente une concentration en nanoparticules de 0,01% à 4% en masse.

3. Utilisation selon l'une quelconque des revendications 1 et 2, dans laquelle le solvant est choisi parmi l'eau ; les solvants organiques tels que les alcools aliphatiques de 1 à 6C, l'acétonitrile, le tetrahydrofuranne, le toluène ; et leurs mélanges.

4. Utilisation selon l'une quelconque des revendications 1 à 3, dans laquelle le sol de nanoparticules est projeté sous la forme d'un jet, nébulisât ou brouillard.

5. Utilisation selon l'une quelconque des revendications 1 à 3, dans laquelle le sol de nanoparticules se présente sous la forme d'un film mince, par exemple d'un film mince recouvrant au moins en partie l'une ou les deux faces d'un substrat plan.

6. Utilisation selon la revendication 5, dans laquelle le film mince du sol de nanoparticules a une épaisseur de 2 nanomètres à 10 micromètres, de préférence de 2 nanomètres à un micromètre.

7. Utilisation selon l'une quelconque des revendications précédentes, dans laquelle les nanoparticules sont des nanoparticules de YₓVO₄Eu₁₋ₓ, où x a une valeur de 0 à 0,995 ; de préférence les nanoparticules sont choisies parmi les nanoparticules de EuVO₄, et les nanoparticules de Y_{0,5}VO₄Eu_{0,5}.

8. Utilisation selon l'une quelconque des revendications précédentes, dans laquelle les nanoparticules ont une taille, par exemple un diamètre, de 2 à 100 nm, de préférence de 10 nm à 20 nm.

9. Utilisation selon l'une quelconque des revendications précédentes, dans laquelle les nanoparticules d'au moins un oxyde d'au moins un métal de transition dopé par une terre rare, sont chacune recouvertes, d'une fine couche de silice obtenue par voie sol-gel ; de préférence la fine couche de silice présente une épaisseur de 1 à 10 nanomètres.

10. Utilisation selon la revendication 9, dans laquelle la silice est fonctionnalisée par des groupes chimiques.

11. Utilisation selon l'une quelconque des revendications précédentes, dans laquelle le capteur est un capteur gravimétrique, de préférence un capteur de microbalance à quartz ; ou un capteur optique à fluorescence.

12. Utilisation selon l'une quelconque des revendications précédentes, dans laquelle le capteur est un multicapteur qui comprend plusieurs capteurs élémentaires choisis parmi les capteurs optiques à fluorescence et les capteurs gravimétriques, au moins un parmi lesdits capteurs élémentaires étant un capteur qui comprend des nanoparticules d'au moins un oxyde d'au moins un métal de transition dopé par une terre rare, obtenues par un procédé sol-gel en tant que matériau sensible ; lesdites nanoparticules se présentant dans le capteur sous la forme d'un sol de nanoparticules dans un solvant.

13. Utilisation selon l'une quelconque des revendications précédentes, dans laquelle le ou les composé(s) ou molécule(s) cible(s) est(sont) choisie(s) parmi les polluants atmosphériques, les explosifs et les précurseurs d'explosifs; de préférence le ou les composé(s) ou molécule(s) cible(s) est(sont) choisie(s) parmi les peroxydes; de préférence encore le ou les composé(s) ou molécule(s) cible(s) est(sont) choisie(s) parmi le peroxyde d'hydrogène, les hydroperoxydes, et les peroxydes de cétones comme le triperoxyde de triacétone et le triperoxyde d'hexaméthylène diamine.

14. Utilisation selon la revendication 13, dans laquelle on réalise la détection ou le dosage sélectif des peroxydes vis-à-vis des composés organiques volatiles.

15. Capteur chimique qui comprend des nanoparticules d'au moins un oxyde d'au moins un métal de transition dopé par une terre rare, obtenues par un procédé sol-gel, en tant que matériau sensible ; dans lequel les nanoparticules se présentent dans le capteur sous la forme d'un sol de nanoparticules dans un solvant.

16. Capteur selon la revendication 15, qui est un capteur gravimétrique, de préférence un capteur à microbalance à quartz ; ou un capteur à fluorescence.

17. Capteur selon l'une quelconque des revendications 15 à 16, qui est destiné à détecter le peroxyde d'hydrogène.

## Patentansprüche

1. Verwendung von Nanopartikeln von wenigstens einem Oxid wenigstens eines Selten-Erden-dotierten Übergangmetalls, hergestellt durch ein Sol-Gel-Verfahren, als sensitives Material in einem chemischen Sensor zur Detektion oder Dosierung von Dämpfen von einer oder mehreren Zielverbindung(en) oder einem oder mehreren Zielmolekül(en) ; bei der sich die Nanopartikel in dem Sensor in Form eines Sols von Nanopartikeln in einem Lösungsmittel präsentieren.

2. Verwendung nach Anspruch 1, bei welcher das Sol von Nanopartikeln eine Massen konzentration von 0.01% bis 4% aufweist.

3. Verwendung nach einem der Ansprüche 1 und 2, bei welcher der Lösungsmittel unter Wasser, die organische Lösungsmittel wie die 1-6C aliphatische Alkoholen, Acetonitril, Tretrahydrofuran, Toluol; und deren Gemischen ausgewählt wird.

4. Verwendung nach einem der Ansprüche 1 bis 3, bei welcher das Sol von Nanopartikeln in Form eines Strahl, einem Dunst, oder einem Nebel, gespritzt wird.

5. Verwendung nach einem der Ansprüche 1 bis 3, bei welcher sich das Sol von Nanopartikeln in Form eines dünnen Films, zum Beispiel eines dünnen Films der wenigstens teilweise eine Seite oder die beiden Seiten eines ebenen Substrats bedeckt, präsentiert.

6. Verwendung nach Anspruch 5, bei welcher der dünne Film des Sols von Nanopartikeln eine Dicke von 2 Nanometern bis 10 Micrometern, vorzugsweise von 2 Nanometern bis ein Micrometer, hat.

7. Verwendung nach einem der vorhergehenden Ansprüche, bei der die Nanopartikel Nanopartikel von YₓVO₄Eu₁₋ₓ sind, wo x einen Wert von 0 bis 0,995 hat; vorzugsweise werden die Nanopartikel ausgewählt unter den Nanopartikeln von EuVO₄, und den Nanopartikeln von Y_{0,5}VO₄EU_{0,5}.

8. Verwendung nach einem der vorhergehenden Ansprüche, bei der die Nanopartikel eine Größe haben, zum Beispiel einen Durchmesser, von 2 bis 100 nm, vorzugsweise von 10 nm bis 20 nm.

9. Verwendung nach einem der vorhergehenden Ansprüche, bei der die Nanopartikel wenigstens eines Oxids von wenigstens einem durch ein Selten-Erden-dotierten Übergangsmetall jede überzogen sind mit einer Siliciumdioxid-Dünnschicht, hergestellt durch das Sol-Gel-Verfahren ; vorzugsweise hat die Siliciumdioxid-Dünnschicht eine Dicke von 1 bis 10 Nanometer.

10. Verwendung nach Anspruch 9, bei der das Siliciumdioxid funktionalisiert wird durch chemische Gruppen.

11. Verwendung nach einem der vorhergehenden Ansprüche, bei welcher der Sensor ein gravimetrischer Sensor ist, vorzugsweise eine Quarzkristall-Mikrowaage Sensor; oder ein optischer Fluoreszenzsensor.

12. Verwendung nach einem der vorhergehenden Ansprüche, bei welcher der Sensor ein Multisensor ist, der mehrere elementare Sensoren umfasst, ausgewählt zwischen den optischen Fluoreszenzsensoren und den gravimetrischen Sensoren, wobei wenigstens einer unter den genannten elementaren Sensoren ein Sensor ist, der Nanopartikel von wenigstens einem Oxid wenigstens eines Selten-Erden-dotierten Übergangsmetalls umfasst, hergestellt durch ein Sol-Gel-Verfahren als sensibles Material; wobei die genanten Nanopartikel in dem Sensor in Form eines Sols von Nanopartikeln in einem Lösungsmittel präsentieren.

13. Verwendung nach einem der vorhergehenden Ansprüche, bei welcher die Zielverbindung(en) oder das (die) Zielmolekül(e) ausgewählt wird (werden) unter den Luftschadstoffen, den Sprengstoffen und den Sprengstoff-Vorprodukten ; vorzugsweise werden die Zielverbindung(en) oder das (die) Zielmolekül(e) ausgewählt unter den Peroxiden, noch bevorzugt die Zielverbindung(en) oder das (die) Zielmolekül(e) ausgewählt unter dem Wasserstoffperoxid; den Hydroperoxiden und den Ketonperoxiden wie dem Triacetontriperoxid und dem Hexamethylendiaminperoxid.

14. Verwendung nach Anspruch 13, bei der man die selektive Detektion oder Dosierung der Peroxide gegenüber den flüchtigen organischen Verbindungen realisiert.

15. Chemischer Sensor, der Nanopartikel von wenigstens einem Oxid wenigstens eines Selten-Erden-dotierten Übergangmetalls umfasst, hergestellt durch ein Sol-Gel-Verfahren, als sensitives Material; bei dem sich die Nanopartikel in dem Sensor in Form eines Sols von Nanopartikeln präsentierten.

16. Sensor nach Anspruch 15, der ein gravimetrischer Sensor ist, vorzugsweise eine Quarzkristall-Mikrowaage Sensor; oder ein Fluoreszenzsensor.

17. Sensor nach einem der Ansprüche 15 bis 16, der dazu bestimmt ist, Wasserstoffperoxid zu detektieren.

## Claims

1. Use of nanoparticles of at least one oxide of at least one transition metal doped with a rare earth element, obtained through a sol-gel process, as a sensitive material in a chemical sensor for detecting or assaying vapors of one or more target compound(s) or molecule(s); in which the nanoparticles are present in the sensor in the form of a sol of nanoparticles in a solvent.

2. Use according to claim 1, in which the sol of nanoparticles has a concentration of nanoparticles of 0.01% to 4% by weight.

3. Use according to any one of claims 1 and 2, in which the solvent is selected from water; organic solvents such as 1 to 6C aliphatic alcohols, acetonitrile, tetrahydrofuran, toluene; and mixtures thereof.

4. Use according to any one of claims 1 to 3, in which the sol of nanoparticles is sprayed in the form of a jet, nebulisate or spray.

5. Use according to any one of claims 1 to 3, in which the sol of nanoparticles is in the form of a thin film, for example a thin film covering at least in part one or both faces of a flat substrate.

6. Use according to claim 5, in which the thin film of the sol of nanoparticles has a thickness of 2 nanometers to 10 micrometers, preferably from 2 nanometers to one micrometer.

7. Use according to any one of the preceding claims, in which the nanoparticles are nanoparticles of YₓVO₄Eu₁₋ₓ, where x has a value of 0 to 0.995; preferably the nanoparticles are selected from nanoparticles of EuVO₄, and nanoparticles of Y₀.₅VO₄Eu_{0.5}.

8. Use according to any one of the preceding claims, in which the nanoparticles have a size, for example a diameter, of 2 to 100 nm, preferably of 10 nm to 20 nm.

9. Use according to any one of the preceding claims, in which the nanoparticles of at least one oxide of at least one transition metal doped with a rare earth element are each covered with a thin layer of silica obtained through a sol-gel process; preferably the thin layer of silica has a thickness of 1 to 10 nanometers.

10. Use according to claim 9, in which the silica is functionalized by chemical groups.

11. Use according to any one of the preceding claims, in which the sensor is a gravimetric sensor, preferably a quartz microbalance sensor ; or a fluorescence optical sensor.

12. Use according to any one of the preceding claims, in which the sensor is a multisensor that comprises several elementary sensors selected from fluorescence optical sensors and gravimetric sensors, at least one among said elementary sensors being a sensor that comprises nanoparticles of at least one oxide of at least one transition metal doped with a rare earth element, obtained through a sol-gel process, as a sensitive material; said nanoparticles being present in the sensor in the form of a sol of nanoparticles in a solvent.

13. Use according to any one of the preceding claims, in which the target compound(s) or molecule(s) is (are) selected from atmospheric pollutants, explosives and precursors of explosives; preferably the target compound(s) or molecule(s) is (are) selected from peroxides; still preferably the target compound(s) or molecule(s) is (are) selected from hydrogen peroxide, hydroperoxides, and peroxides of ketones such as triacetone triperoxide and hexamethylene diamine triperoxide.

14. Use according to claim 13, in which the selective detection or assaying of peroxides vis-à-vis volatile organic compounds is carried out.

15. Chemical sensor that comprises nanoparticles of at least one oxide of at least one transition metal doped with a rare earth element, obtained through a sol-gel process, as a sensitive material; in which the nanoparticles are present in the sensor in the form of a sol of nanoparticles in a solvent.

16. Sensor according to claim 15, which is a gravimetric sensor, preferably a quartz microbalance sensor ; or a fluorescence sensor.

17. Sensor according to any of claims 15 to 16, which is intended to detect hydrogen peroxide.
